# EUROPEAN PATENT SPECIFICATION

(11) **EP 1 502 146 B1**
(45) Date of publication and mention of the grant of the patent: **05.07.2006**
(21) Application number: 02806423.6
(22) Date of filing: 09.04.2002
(51) Int. Cl.: G02B 27/14, A42B 3/26, A61F 9/02

(54) **TEAR-OFF OPTICAL STACK HAVING PERIPHERAL SEAL MOUNT**
OPTISCHER ABREISSSTAPEL MIT UMFANGSVERSIEGELUNGSANBRINGUNG
EMPILEMENT OPTIQUE DETACHABLE AYANT UNE MONTURE PERIPHERIQUE HERMETIQUE

(30) Priority: 03.01.2002 US 39519
(43) Date of publication of application: 02.02.2005
(73) Proprietor: Wilson, Stephen S., San Juan Capistrano, CA 92675 (US)
(72) Inventor: WILSON, Stephen, S., San Juan Capistrano, CA 92675 (US); WILSON, Seth, San Juan Capistrano, CA 92675 (US); WILSON, Bart, San Juan Capistrano, CA 92675 (US)
(74) Representative: Gervasi, Gemma
(86) International application number: PCT/US2002/010971
(87) International publication number: WO 2003/060563

(56) References cited:
- GB-A- 2 269 239
- US-A- 2 511 329
- US-A- 4 076 373
- US-A- 4 138 746
- US-A- 4 716 601
- US-A- 5 592 698
- US-A- 5 671 483
- US-A- 6 085 358
- US-B1- 6 388 813

## Description

### BACKGROUND OF THE INVENTION

The present invention generally relates to the field of eye protectors or face shields having a lens cover plate, and in particular to peripheral seal mounts for a tear-off stack of laminated removable lens covers adhered to face shields and the like.

It is common practice to wear face shields in environments where injury of the eyes or face may occur from contamination. For example, face shields are worn by physicians working in the operating room. They are sometimes worn by those playing games where injury to the eyes may occur. For example, in the game of Paint Ball contestants employ weapons that fire at one another pellets or balls of paint as ammunition. When a ball of paint strikes a participant, it becomes readily apparent that they have been hit by a "tell-tale" blotch of paint. Obviously, it is desirable for contestants to wear some form of protection over their face and eyes.

In such environments as the Paint Ball game or a the operating room, it is most likely that any face protection lens being worn will become covered with contaminants, which obscures the view. The solution would appear to be to simply clean off the lens. This is not always an option, especially for a physician performing an operation who cannot stop for such cleaning. An example of a recent solution to a similar problem is the use of a multi-ply transparent lens over goggles in order to facilitate rapid removal of dirt or grime in contaminated environments. That is, when the goggles become contaminated, a single layer of the multi-ply transparent lens is removed. The goggles are now clean and when they again become contaminated another layer of the multi-ply transparent lens is removed. This process can be repeated many times, depending upon the number of layers applied to the goggles. An example of this process and the materials used is disclosed in our pending application serial no. 09/449,318 filed November 24, 1999 entitled OPTICAL STACK OF LAMINATED REMOVABLE LENSES FOR FACE SHIELDS, WINDOWS, AND DISPLAYS, published as US 6388813.

In our above-cited patent, the problem we overcame was caused by reduced visibility as a result of the additive effect of the optical index of refraction for each layer applied. We overcame this problem by eliminating the air space between each successive layer of the multi-ply transparent lens and by inserting an adhesive layer between each lens layer. Thus, we created an adhesively laminated multi-layered clear film adapted to be used on a wearer's goggles, face shield or the like. However, when this same film is used for large area face protectors other problems arise. For example, when the bottom-most layer is adhered directly to the lens bubbles will appear, which bubbles are not easily removed because of the multi-layers over the bottom-most layer. Moreover, if the adhesive is omitted from the bottom-most layer, then we are back to the original refraction problem of an air gap between the eye-protector lens and the bottom-most layer of the multi-ply transparent lens. In addition, if the adhesive layer is removed from the interface between the bottom-most layer of the multi-ply stack and the lens of the face protector, then there remains the problem with the lens fogging up in the presence of moisture.

Accordingly, there is a need for a multi-ply transparent lens that will not produce bubbles when applied to the face protector's lens and will not reduce visibility due to a fogging up of the lens by moisture seeping into the interface between the bottom-most layer of the removable lens and the lens of the eye protector.

### BRIEF SUMMARY OF THE INVENTION

Accordingly, a feature of the present invention is the use of selective areas of adhesive around the periphery of the bottom-most layer of a multi-ply transparent optical lens stack for attachment to a large area face protector.

Another feature of the present invention is a method for the removal of a peripheral race around the edges of the bottom-most layer of a multi-ply transparent lens stack, thereby exposing adhesive around the periphery only. The peripheral adhesive area may then be used for affixing the multi-layer transparent lens to the face protector. Thus, the central area of the bottom-most layer of the multi-ply transparent lens remains in place, thereby avoiding the bubble problem. Also, the adhesive on the periphery solves the fogging up problem by sealing out any moisture.

Yet another feature of the present invention is an alternate method of forming the adhesive strip by applying a thin strip of adhesive directly to the bottom-most lens of the optical stack around the periphery thereof.

Still another feature of the present invention is the provision of an optical stack of removable lenses that may be used for windshields of vehicles, windows of all types, or video displays.

These and other features, which will become apparent as the invention is described in detail below, are provided by an optical stack of laminated removable lenses that may be affixed to a face shield. The stack includes a plurality of superposed removable lenses adhesively affixed to one another, wherein each of the removable lenses is held to each successive lens with a clear uninterrupted adhesive layer interposed between each lens. Each of the lens has a removal tab portion on one end which does not have any adhesive layer on either side thereof. This allows the wearer of the face shield to quickly grasp the removal tab portion for removing the top lens and exposing a clean lens directly underneath. The bottom-most removable lens of the stack has a band of adhesive formed around the periphery thereof for attachment of the stack to the face shield. The band of adhesive forms a seal which eliminates any moisture from the interface between the bottom-most lens and the shield.

Still other features and advantages of the present invention will become readily apparent to those skilled in the art from the following detailed description, wherein is shown and described only the preferred embodiment of the invention, simply by way of illustration of the best mode contemplated of carrying out the invention. As will be realized, the invention is capable of other and different embodiments, and its several details are capable of modifications in various obvious respects, all without departing from the invention. Accordingly, the drawings and description are to be regarded as illustrative in nature, and not as restrictive, and what is intended to be protected by Letters Patent is set forth in the appended claims.

### BRIEF DESCRIPTION OF THE DRAWINGS

The general purpose of this invention, as well as a preferred mode of use, its objects and advantages will best be understood by reference to the following detailed description of an illustrative embodiment with reference to the accompanying drawings in which like reference numerals designate like parts throughout the figures thereof, wherein:
Figure 1 is a perspective view of a face protector that may employ the multi-ply transparent lens stack according to the teachings of the present invention.
Figure 2 is a perspective view of a face protector having attached thereto the multi-ply transparent optical stack according to the teachings of the present invention.
Figure 3 is a partially exploded view of the multi-ply transparent optical stack showing the peripheral cut in the bottom-most layer, which cut exposes a periphery of adhesive for application to the face protector shown in Figure 2.
Figure 4 is a partial cross-sectional view taken along line 4-4 in Figure 3 showing a detail of the alternating film layers with adhesive layers between the film layers.

### DETAILED DESCRIPTION OF THE INVENTION

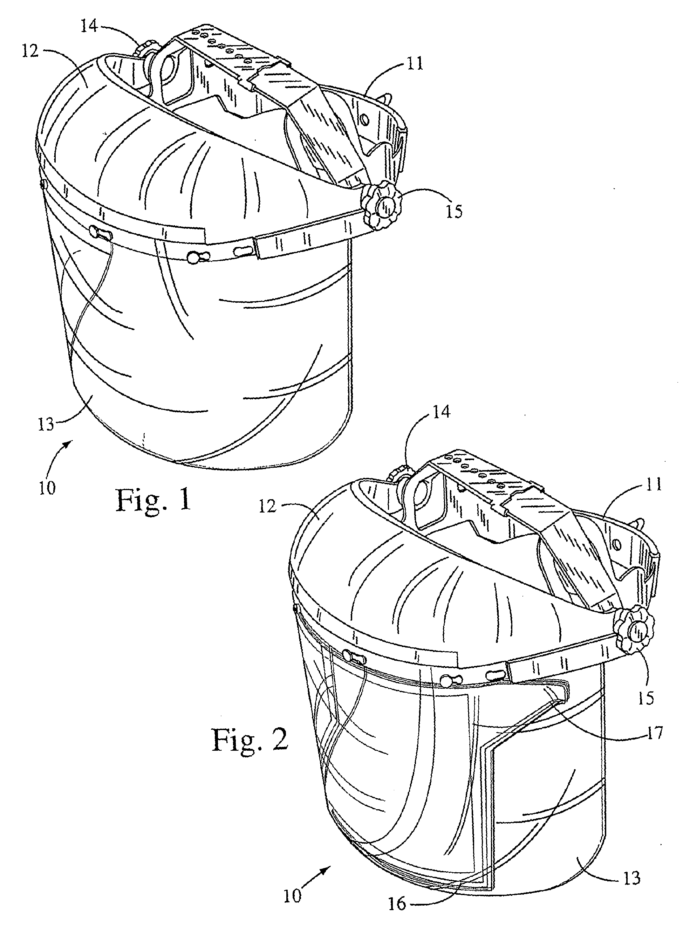

Referring now to the drawings and Figure 1 in particular, a large area face protector 10 is shown in perspective view. The face protector may include an adjustable headband 11 and a frontal member 12 for supporting a lens 13. The frontal member 12 and lens 13 may be adjusted at an angle with the headband 11. Adjustment knobs 14 and 15 are available for holding the member 12 and lens 13 at the desired angle. Although a particular face protector is depicted, those skilled in the art will recognize that all face protectors, goggles and the like are contemplated for use in the present invention.

Referring now to Figure 2, a perspective view of the face protector 10 is shown with a multi-ply transparent lens optical stack 16 attached thereto. The lens optical stack 16 is made and attached to the lens 13 according to the teachings of the present invention, amplified herein below. The lens optical stack 16 preferably includes tabs 17, which are employed by a wearer for removal of an individual layer of the lens optical stack 16. It is preferable that the removal tabs 17 do not have any adhesive on either side thereof so that a user will not pick up the adhesive on their fingers or gloves (if worn).

During use for example, when the lens optical stack 16 becomes contaminated and blocks the user's view, then one simply reaches up and grabs an individual tab 17 and peels off an outer layer of the lens optical stack 16. This process may be repeated as many times as there are layers in the lens optical stack 16.

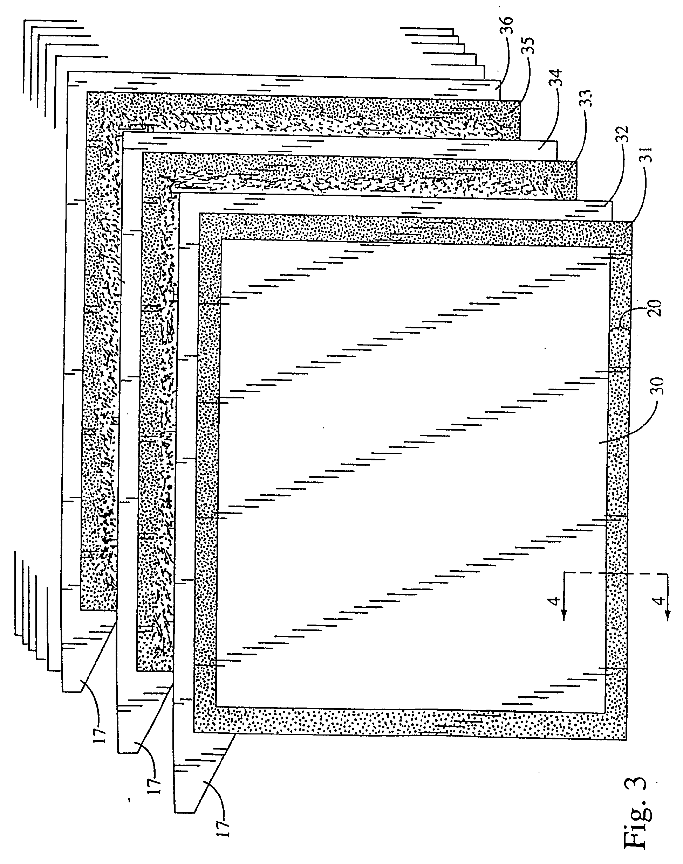

Referring now to Figure 3, a partially exploded view of the multi-ply transparent optical stack 16 is shown. This figure illustrates removable Mylar lenses or film layers 30, 32, 34, 36 ... alternating with adhesive layers 31, 33, 35 .... That is, the film layer 30 is held to the film layer 32 by the clear uninterrupted adhesive layer 31 interposed there between. The next film layer 34 is held to the film layer 32 by interposing the adhesive layer 33 there between, and so forth for formation of the entire lens optical stack 16.

The preferred material used to form the film layers 30, 32, 34, etc. is preferably a clear polyester. The layers are preferably fabricated from sheets of plastic film sold under the registered trademark Mylar owned by the DuPont Company and more particularly a type of Mylar made from a clear polymer polyethylene terephalate, commonly referred to as PET. The preferred adhesive layers 31, 33, 35 etc. used to laminate the lenses together sequentially is a clear optical low tack material. The thickness of each film layer typically ranges from .5 mil to 7 mil (1 mil is .001"). The preferred thickness is 2 mil. Even after the adhesive material is applied to a 2 mil thickness layer, the thickness of the 2 mil thickness layer will still be 2 mil due to the adhesive film layer having only a nominal thickness. The term "wetting" can be used to describe the relationship between the laminated film layers. When viewing through the laminated layers, it appears to be one single piece of plastic film. No reflections are evident. The adhesive material 20 comprises a water based acrylic optically clear adhesive or an oil based clear adhesive, with the water based adhesive being the preferred embodiment. After the film layers 30, 32, 34 etc. are laminated or otherwise bonded together with the interposed adhesive layers 31, 33, 35 etc., the thickness of each adhesive layer is negligible even though the adhesive layers are illustrated in as distinct layers. A preferred adhesive layer/film layer construction is disclosed in our pending application serial no. 09/449,318 filed November 24, 1999 entitled Optical Stack of Laminated Removable Lenses for Face Shields, Windows, and Displays, published as US6388813.

In accordance with a first embodiment of the present invention, a cut is made around the periphery of the bottom-most film layer 30, thereby exposing a narrow strip 20 of the adhesive layer 31. The strip 20 is used for adhesively affixing the optical stack 16 to the face protector 10. It is preferable to make the peripheral cut after the optical stack 16 is assembled into the multiple alternating layers in order to maintain proper registration of the individual layers. However, those skilled in the art will recognize the narrow strip 20 may be applied separately to the bottom most film layer 30 as well.

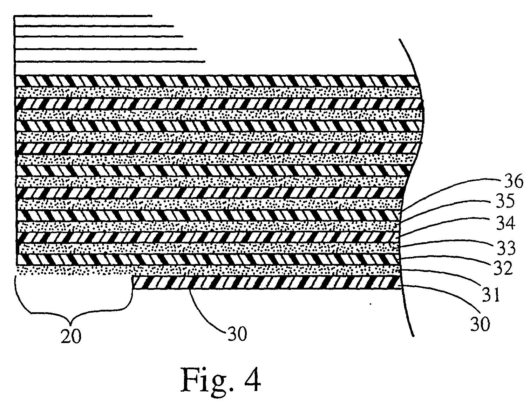

Referring now to Figure 4, a partial cross-sectional view taken along line 4-4 of Figure 3 illustrates a detail of alternating film layers 30, 32, 34, 36 ..., with the adhesive layers 31, 33, 35, ... interposed between each the film layers. It is noted that the peripheral cut exposing the strip 20 only removes a portion of the bottom-most film layer 30. This narrow peripheral strip 20 is preferably ⅛" to ½" in width, or wider. The more narrow the strip the more likely the stack will not stay in place on the face protector and the wider the strip the more likely the bubble problem, discussed above, will occur. Hence, I have discovered that the preferred width is as stated above, i.e., ⅛" to ½" in width.

As will be recognized, the multiply transparent optical stack 16 of the present invention can be easily applied to any desired face protector 10 by positioning the bottom-most film layer 30 having the narrow strip 20 of adhesive layer disposed about the periphery thereof adjacent to the front surface of the face protector. A user may then press the narrow strip 20 of the adhesive layer 31 against the outer surface of the face protector 10 causing the stack to be rapidly mounted thereto. In the preferred embodiment, the narrow strip 20 is attached to the face protector 10 adjacent one end thereof and subsequently spread or pressed tightly against the front surface of the face protector 10. In view of the large optical area of bottom-most film layer 30 being devoid of any adhesive, air bubbles or the like are not presented at the interface of the optical stack 16 and the face protector 10. However, because the optical stack 16 has adhesive layers there between, reflection between the film layers 30 is substantially reduced. During use, as unwanted material is splashed or presented to upper most film layer, the user may quickly remove the upper most film layer from the stack exposing a clean film layer there below. As such, it will be recognized that the present invention allows an effective optical stack to be rapidly deployed upon any optical surface and be rapidly used by a user.

Although the invention has been described with reference to a specific embodiment, this description is not meant to be construed in a limiting sense. Various modifications of the disclosed embodiment as well as alternative embodiments of the invention will become apparent to one skilled in the art upon reference to the description to the invention. It is therefore contemplated that the appended claims will cover any modifications of the embodiments that fall within the true scope of the invention.

## Claims

1. An optical stack (16) of laminated removable lenses for adhering to a face shield comprising:
(a) a plurality of superposed removable lenses (30,32,34,36,...) adhesively affixed to one another;
(b) each said removable lens being held to each successive lens with a clear uninterrupted adhesive layer (31,33,35,...) interposed between each said removable lens;
(c) each said lens having a removal tab portion (17) on at least one end which does not have any adhesive layer on either side of said tab portion for allowing the wearer of the face shield to quickly grasp said removal tab portion for removing the top lens and exposing a clean lens directly underneath said removed top lens; **characterized by**:
(d) the bottom-most removable lens (30) of said stack having a band (20) of adhesive formed around the periphery thereof for attachment of said stack to said face shield, wherein said band of adhesive forms a seal thereby eliminating any moisture from the interface between said bottom-most lens and said shield.

2. The optical stack as in Claim 1 wherein said band (20) of adhesive is formed by making a cut to the depth of the bottom-most lens (30), inside the periphery and contiguous to the lens shape, whereby the outer most generally annulus portion is then removed, thereby exposing the adhesive of the previous layer, which aids in mounting said lens to said face shield.

3. The optical stack as in Claim 1 wherein said band (20) of adhesive is applied to the periphery of said bottom-most lens.

4. The optical stack as in Claim 1 wherein said removable lenses and said clear adhesive layers have an index of refraction between 1.40 and 1.52.

5. An optical stack (16) of removable lenses for adhering to an optical window comprising:
a) a plurality fo generally rectangular superposed removable lenses (30,32,34,36,...) adhesively adhered to one another so as to form said optical stack;
b) each said removable lens being held to each successive lens with a clear uninterrupted adhesive layer (31,33,35,...) interposed between each said removable lens;
c) a plurality of staggered tabs (17) extending from a corner of each of said film layers, said tabs being disposed to assist in allowing each film layer to be peeled off successively; and
d) the bottom-most removable lens (30) of said stack having a band (20) of adhesive formed around the periphery thereof for attachment of said stack to said optical window, wherein said band of adhesive forms a seal thereby eliminating any moisture from the interface between said bottom-most lens and said optical window.

6. The optical stack as in Claim 5 wherein the optical window to which said stack of removable lenses is mounted includes a windshield, window, face shield or a video display.

7. The optical stack as in Claim 5 wherein said band (20) of adhesive is formed by making a cut to the depth of the bottom-most lens (30), inside the periphery and contiguous to the lens shape, whereby the outer most generally annulus portion is then removed, thereby exposing the adhesive of the previous layer, which aids in mounting said lens to said optical window.

8. The optical stack as in Claim 5 wherein said band (20) of adhesive is applied to the periphery of said bottom-most lens (30).

9. The optical stack as in Claim 5 wherein said removable lenses and said clear adhesive layers have an index of refraction between 1.40 and 1.52.

10. The optical stack as in Claim 5 wherein said tabs (17) do not have adhesive attached thereto.

11. A method for forming an optical lens stack (16) for attachment to an optical window, said method comprising the steps of:
a) laminating together a multilplicity of optical lens layers (30,32,34,36,...) while interposing a clear uninterrupted adhesive layer (31,33,35,...) between each optical lens layer being laminated;
b) forming a tab extension (17) from each optical lens layer and omitting said adhesive layer from said tab extension, whereby each of said lens layer may be peeled off individually; and,
c) cutting around the perimeter and to a depth of one optical lens ply the bottom-most layer (30) of said optical stack, thereby exposing a strip (20) of adhesive for adhering said optical stack (16) to said optical window.

## Patentansprüche

1. Optischer Stapel (16) aus laminierten abziehbaren Linsen zum Aufkleben auf einem Gesichtsschutzschild, welcher umfasst:
(a) eine Vielzahl von übereinander gestapelten abziehbaren Linsen (30,32,34,36,...), welche klebend aneinander gefügt sind;
(b) wobei jede abziehbare Linse an jeder nachfolgenden Linse mit einer durchsichtigen ununterbrochenen Klebeschicht (31,33,35,...) befestigt ist, welche zwischen jeder abziehbaren Linse aufgebracht ist;
(c) wobei jede Linse einen Abziehlaschenabschnitt (17) an zumindest einem Ende aufweist, und wobei der Abziehlaschenabschnitt auf beiden Seiten keine Klebeschicht aufweist, um den Träger des Gesichtsschutzschilds zu ermöglichen, schnell den Abziehlaschenabschnitt zu greifen, zum Abziehen der obersten Linse, und zum Freilegen einer sauberen Linse direkt unterhalb der abgezogenen obersten Linse; **gekennzeichnet durch**:
(d) eine unterste abziehbare Linse (30) des Stapels mit einem Klebeband (20), ausgebildet entlang des Umfangs der Linse zum Befestigen des Stapels an dem Gesichtsschutzschild, wobei das Klebeband eine Dichtlippe bildet, und **dadurch** jede Feuchtigkeit von der Verbindungsfläche zwischen der untersten Linse und dem Gesichtsschutzschild fernhält.

2. Optischer Stapel gemäß Anspruch 1, wobei das Klebeband (20) gebildet wird, indem ein Schnitt in die Tiefe der untersten Linse (30) eingebracht wird, innerhalb des Umfangs und entlang der Linsenform, wodurch der äußerste, gewöhnlich ringförmige Abschnitt dann abgetrennt wird, und wobei dadurch der Kleber der vorherigen Schicht freigelegt wird, welcher dazu beiträgt, die Linse an dem Gesichtsschutzschild zu befestigen.

3. Optischer Stapel gemäß Anspruch 1, wobei das Klebeband (20) an dem Umfang der untersten Linse angebracht ist.

4. Optischer Stapel gemäß Anspruch 1, wobei die abziehbaren Linsen und die durchsichtigen Klebeschichten einen Brechungsindex zwischen 1,4 und 1,52 aufweisen.

5. Optischer Stapel (16) aus abziehbaren Linsen zum Aufkleben auf einem optischen Fenster, welcher umfasst:
(a) eine Vielzahl von gewöhnlich rechteckigen, übereinander gestapelten, abziehbaren Linsen (30,32,34,36,...), welche klebend aneinander gefügt sind, um den optischen Stapel zu bilden;
(b) wobei jede abziehbare Linse an jeder nachfolgenden Linse mit einer durchsichtigen ununterbrochenen Klebeschicht (31,33,35,...) befestigt ist, welche zwischen jeder abziehbaren Linse aufgebracht ist;
(c) eine Vielzahl von versetzt angeordneten Laschen (17), welche von einer Ecke von jeder der Filmschichten abstehen, wobei die Laschen zur Unterstützung angebracht sind, um zu ermöglichen, dass jede Filmschicht nacheinander abgezogen wird; und
(d) wobei die unterste abziehbare Linse (30) des Stapels ein Klebeband (20) aufweist, welches entlang des Umfangs davon ausgebildet ist, um den Stapel an dem optischen Fenster zu befestigen, wobei das Klebeband eine Dichtlippe bildet, und dadurch Feuchtigkeit von der Verbindungsfläche zwischen der untersten Linse und dem optischen Fenster fernhält.

6. Optischer Stapel gemäß Anspruch 5, wobei das optische Fenster, an welchem der Stapel aus abziehbaren Linsen befestigt ist, umfasst, ein Windschutzschild, ein Fenster, ein Gesichtsschutzschild oder ein Videodisplay.

7. Optischer Stapel gemäß Anspruch 5, wobei das Klebeband (20) ausgebildet wird, indem ein Schnitt in die Tiefe der untersten Linse (30) eingebracht wird, innerhalb des Umfangs und entlang der Linsenform, wobei der äußerste, gewöhnlich ringförmige Abschnitt dann entfernt wird, und wobei dadurch der Klebstoff der vorherigen Schicht freigelegt wird, welcher dazu beiträgt, die Linse an dem optischen Fenster zu befestigen.

8. Optischer Stapel gemäß Anspruch 5, wobei das Klebeband (20) an dem Umfang der untersten Linse (30) angebracht ist.

9. Optischer Stapel gemäß Anspruch 5, wobei die abziehbaren Linsen und die durchsichtigen Klebeschichten einen Brechungsindex zwischen 1,4 und 1,52 aufweisen.

10. Optischer Stapel gemäß Anspruch 5, wobei die Laschen (17) keinen darauf aufgebrachten Klebstoff aufweisen.

11. Verfahren zum Bilden eines Stapels (16) aus optischen Linsen zur Befestigung an einem optischen Fenster, wobei das Verfahren die nachfolgenden Schritte umfasst:
(a) das Aufeinanderlaminieren einer Vielzahl von Schichten (30,32,34,36,...) aus optischen Linsen, indem eine durchsichtige, ununterbrochene Klebeschicht (31,33,35,...) zwischen jede Schicht aus einer optischen Linse, welche laminiert wird, aufgebracht wird;
(b) das Bilden einer Laschenerweiterung (17) bei jeder Schicht aus einer optischen Linse, und das Weglassen der Klebeschicht bei der Laschenerweiterung, wobei jede Linsenschicht einzeln abgezogen werden kann; und
(c) das Schneiden entlang dem Umfang und in die Tiefe einer Lage aus einer optischen Linse der untersten Schicht (30) des optischen Stapels, wobei dadurch ein Klebestreifen (20) freigelegt wird, um den optischen Stapel (16) an dem optischen Fenster anzukleben.

## Revendications

1. Un empilement optique (16) de lentilles laminées détachables pour l'adhésion à un écran frontal comprenant :
(a) une multitude de lentilles détachables (30, 32, 34, 36,....) superposées, fixées de façon adhésive l'une à l'autre ;
(b) chacune desdites lentilles détachables étant maintenue à chacune des lentilles suivantes avec une couche (31, 33, 35,..) continue incolore, interposée entre chacune desdites lentilles détachables ;
(c) chacune desdites lentilles ayant une partie (17) de languette détachable sur au moins une extrémité dépourvue de toute couche adhésive sur les deux côtés de ladite partie de languette pour permettre au porteur de l'écran frontal de saisir rapidement ladite partie de languette détachable afin de retirer les lentilles supérieures et de présenter une lentille propre directement sous ladite lentille supérieure détachée ; **caractérisé en ce que** :
(d) la lentille (30) détachable située la plus en bas dudit empilement, a une bande (20) d'adhésif formée autour de sa périphérie pour attacher ledit empilement audit écran frontal ; ladite bande d'adhésif formant une étanchéité qui élimine toute humidité de l'interface entre ladite lentille située le plus en bas et ledit écran.

2. L'empilement optique selon la revendication 1, où ladite bande (20) d'adhésif est formée en réalisant une coupure dans l'épaisseur de la lentille (30) située le plus en bas, à l'intérieur de la périphérie et contiguë à la forme de la lentille ; la partie généralement annulaire située le plus à l'extérieur étant alors retirée, exposant ainsi l'adhésif de la couche précédente qui offre une aide au montage de ladite lentille audit écran frontal.

3. L'empilement optique selon la revendication 1, où ladite bande (20) d'adhésif est appliquée à la périphérie de ladite lentille située la plus en bas.

4. L'empilement optique selon la revendication 1, où ladite lentille détachable et lesdites couches d'adhésifs incolores ont un index de réfraction situé entre 1,4 et 1,52.

5. Un empilement optique (16) selon la revendication 1de lentilles détachables pour l'adhésion à une fenêtre optique, comprenant :
(a) une multitude de lentilles (30, 32, 34, 36,...) détachables, superposée généralement de façon rectangulaire, collées l'une à l'autre afin de former ledit empilement optique ;
(b) chacune desdites lentilles détachables étant maintenues à chacune des lentilles suivantes avec une couche (31, 33, 35, ...) avec une couche adhésive continue incolore, interposée entre chacune desdites lentilles détachables ;
(c) une multitude de languettes (17) décalées s'étendant à partir d'un coin de chacune desdites couches en forme de film, lesdites languettes étant disposées pour permettre à chaque couche en forme de film d'être retirées successivement ; et
(d) ladite lentille (30) détachable située le plus en bas dudit empilement ayant une bande (20) d'adhésif formée autour de ladite lentille pour attacher ledit empilement à ladite fenêtre optique ; ladite bande d'adhésif formant une étanchéité éliminant ainsi toute humidité de l'interface entre la lentille située le plus en bas et ladite fenêtre optique.

6. L'empilement optique selon la revendication 5, où la fenêtre optique sur laquelle est montée ledit empilement de lentilles détachables, comporte un écran contre le vent, une fenêtre, un écran frontal ou un affichage vidéo.

7. L'empilement optique selon la revendication 5, où ladite bande (20) d'adhésif est formée en réalisant une coupure dans la profondeur de la lentille (30) située le plus en bas, à l'intérieur de la périphérie et contiguë à la forme de la lentille ; la partie généralement annulaire située le plus à l'extérieur étant alors retirée, exposant ainsi l'adhésif de la couche précédente qui offre une aide au montage de ladite lentille audit écran frontal.

8. L'empilement optique selon la revendication 5, où ladite bande (20) d'adhésif est appliquée sur la périphérie de ladite lentille (30) située le plus en bas.

9. L'empilement optique selon la revendication 5, où lesdites lentilles détachables et lesdites couches d'adhésifs incolores ont un index de réfraction situé entre 1.40 et 1.52.

10. L'empilement optique selon la revendication 5, où aucun adhésif n'est fixé sur lesdites languettes (17).

11. Un procédé pour la formation d'un empilement (16) de lentilles optiques destiné à être attaché à une fenêtre optique, ledit procédé comprenant les étapes suivantes :
a) laminage commun d'une multitude de couches (30, 32, 34, 36, ...) de lentilles optiques en interposant une couche (31, 33, 35,...) d'adhésif continue incolore entre chacune des couches de lentilles optiques ayant été laminées ;
b) la formation d'une extension (17) de languette à partir de chacune des couches de lentilles optiques et l'omission de ladite couche adhésive de ladite extension en forme de languette ; chacune desdites couches de lentilles pouvant être retirées individuellement ; et
c) le découpage autour du périmètre et de la couche (30) située le plus en bas, dans la profondeur d'un pli de lentille optique dudit empilement optique, exposant ainsi une bande (20) d'adhésif destiné à coller ledit empilement (16) optique à ladite fenêtre optique.
